(19) Europäisches Patentamt
European Patent Office
Office européen des brevets

(11) Publication number: 0 168 505 B1

# (12) EUROPEAN PATENT SPECIFICATION

(45) Date of publication of patent specification: 09.08.89

(21) Application number: 84105843.1

(22) Date of filing: 22.05.84

(51) Int. Cl.[4]: C 07 D 333/20, C 07 D 307/52, C 07 D 213/38, C 07 D 207/32, C 07 D 209/08, C 07 D 233/54, C 07 C 91/28, C 07 C 93/26, C 07 C 121/66, A 61 K 31/38, A 61 K 31/135

## (54) Substituted 2-aminotetralins and processes for synthesis.

(43) Date of publication of application:
22.01.86 Bulletin 86/04

(45) Publication of the grant of the patent:
09.08.89 Bulletin 89/32

(84) Designated Contracting States:
BE CH DE FR GB IT LI NL SE

(56) References cited:
EP-A-0 026 848
EP-A-0 041 488
EP-A-0 064 964

JOURNAL OF MEDICINAL CHEMISTRY, vol. 22, no. 12, December 1979, pages 1469-1475; U. HACKSELL et al.: "N-alkylated 2-aminotetralins: Central dopamine-receptor stimulating activity"

The file contains technical information submitted after the application was filed and not included in this specification

(73) Proprietor: NELSON RESEARCH & DEVELOPMENT COMPANY
1001 Health Sciences Road West
Irvine, CA 92715 (US)

(72) Inventor: Horn, Alan S.
Noordwijkweg 3A
Noordhorn (GR) (NL)

(74) Representative: Kraus, Walter, Dr. et al
Patentanwälte Kraus, Weisert & Partner
Thomas-Wimmer-Ring 15
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 168 505 B1

**Description**

The invention relates generally to substituted 2-aminotetralins and to processes for preparing such compounds. More particularly, the invention relates to compounds and methods for preparing the compounds, which are useful in particular in treating disorders of the central nervous, cardiovascular and endocrine systems.

It is known that various hydroxylated 2-aminotetralins of the general formula

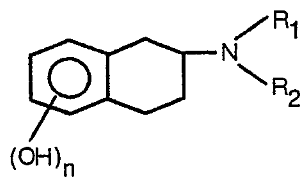

where $R_1$ and $R_2$ are saturated alkyl groups and n is 1 or 2, are dopamine receptor agonists (Mc Dermed et al., J. Med. Chem. *18*, 362, 1975; Feenstra et al., Arch. Pharmacol. *313*, 213, 1980).

Journal of Medicinal Chemistry, Vol. 22, No. 12, December 1979, pages 1469 to 1475 describes specific N-alkylated-2-aminotetralins having a central dopamine-receptor stimulating activity e.g. 5-hydroxy-2-(N-n-propyl-N-2-phenylethyl)aminotetralin. EP—A—0 064 964, EP—A—0 041 488 and EP—A—0 026 848 disclose effective dopamine receptor agonists having the structure of a substituted 2-(N-n-propyl)aminotetralin.

The present invention provides new compounds having the structural formula

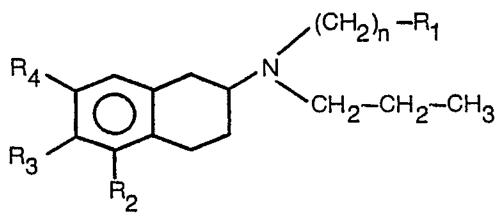

wherein $R_2$, $R_3$ and $R_4$ are each selected from H, and OA; A is H or

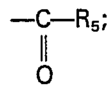

$R_5$ is selected from alkyl and aromatic residues; n is 2 or 3; and $R_1$ is selected from the group consisting of 3-pyridyl, 4-pyridyl,

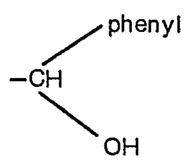

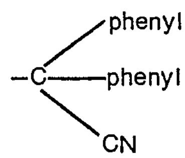

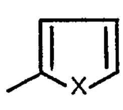 , 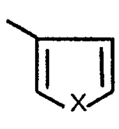 , 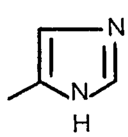 and 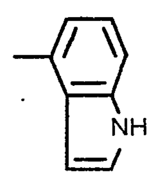

wherein X is S, O or NH, and a pharmaceutically acceptable salt thereof, with the proviso that at least one of the $R_2$, $R_3$ and $R_4$ is H, that at least one of $R_2$, $R_3$ and $R_4$ is not H and that $R_2$ and $R_4$ are not both OA.

The compounds are useful as dopamine receptor agonists for the treatment of disorders of the central nervous, cardiovascular and endocrine systems such as Parkinson's disease and related disorders, hypertension and hyperprolactinemia.

The invention also provides a process for the production of the above-described compounds comprising the steps of

(a) condensing a β-tetralone of the formula

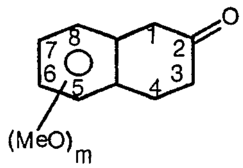

with a primary amine of the formula $R_{\emptyset}$—$(CH_2)_n$—$NH_2$ wherein m is 1 or 2, and if n is 3 then $R_{\emptyset}$ is $R_1$, and if n is 2 then $R_{\emptyset}$ is $CH_3$ or $R_1$;

(b) reducing the product of step (a) to form a secondary amine of the formula

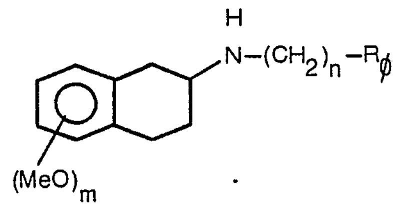

(c) reacting the product of step (b) either with a compound of the formula $R_6$—$(CH_2)_p$—Y in the presence of a base or with a compound of the formula $R_6$—$(CH_2)_o$—COOH in the presence of a reducing agent to form a tertiary amine, or with a compound of the formula

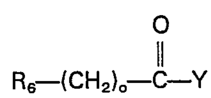

to form an amide and reducing the amide to a tertiary amine; in the above formulae Y is Cl, Br, I, tysolate, or mesylate; and if $R_{\emptyset}$ is $R_1$ then $R_6$ is $CH_3$, o is 1, and p is 2; and if $R_{\emptyset}$ is $CH_3$ then $R_6$ is $R_1$, o is 1 or 2, and p is 2 or 3; where the tertiary amine has the formula:

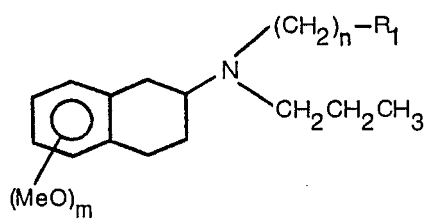

and

(d) demethylating the ether linkages of the tertiary amine to form the desired product.

We have also discovered a process for synthesizing esters of the foregoing synthesized compounds by reacting the compounds with a carboxylic acid chloride.

The compounds described above may be prepared by the general methods outlined below. The numeral references in parenthesis following intermediates refer to numbered structural formulas below.

The esters and acid addition salts of the compounds of the general formula are prepared in the conventional manner. As acid addition salts can be used the salts derived from a therapeutically acceptable acid such as hydrochloric acid, acetic acid, propionic acid and, more particularly, from a di- or polybasic acid such as phosphoric acid, succinic acid, maleic acid, fumaric acid, citric acid, glutaric acid, citraconic acid, glutaconic acid, tartaric acid, malic acid, and ascorbic acid.

Method I

The β-tetralone (1) is condensed with a primary amine in the presence of an acid catalyst such as TsOH. The resulting intermediate is then reduced (e.g. with $H_2/PtO_2$, $NaBH_3CN$ etc.) to yield the secondary amine (2). This is then acylated with propionyl chloride to give the amide (3). This amide is then reduced to the tertiary amine and the latter is demethylated with HBr or $BBr_3$ (depending on the nature of group $R_1$) to yield the phenol or catechol of general structure (4).

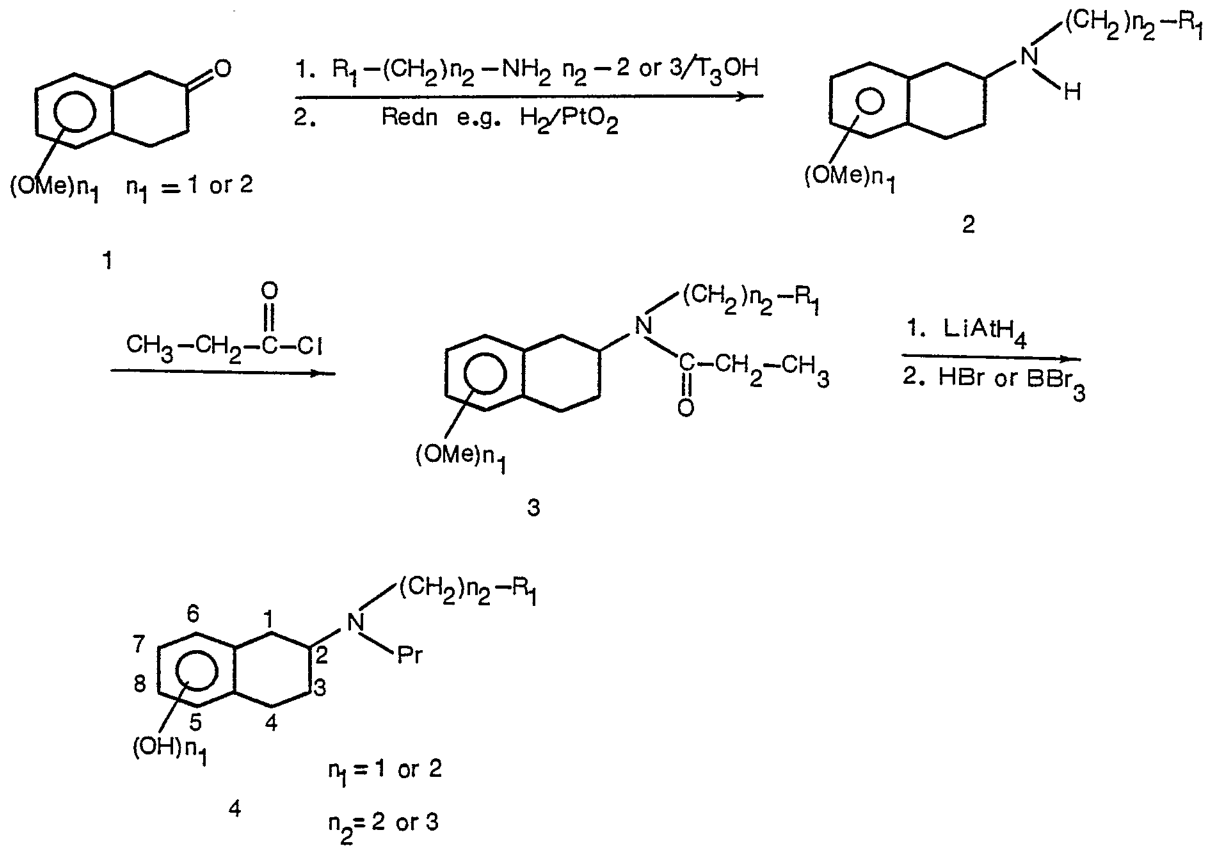


1 2 3 4

Method II

Compound (5) is prepared by known methods such as condensation with propylamine followed by reduction. The intermediate (5) can be converted to compound (6) in two ways:

A. via acylation with an acid chloride followed by reduction with $LiAlH_4$ or

B. via direct alkylation with the appropriate alkyl halide. Compound (4) is prepared from compound (6) by treatment with HBr or $BBr_3$.

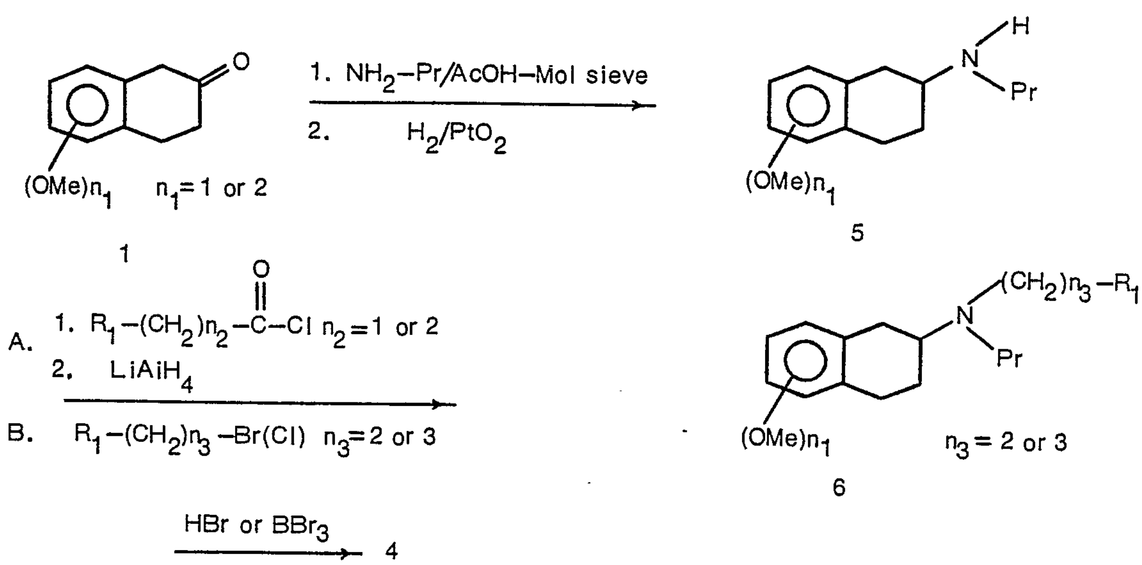


1 5 6

Method III

Compound (5) is treated with the appropriate carboxylic acid and sodium borohydride to compound (6) in one step. (c.f. Hacksell et al., J. Med. Chem. *22*, 1469, 1979). Compound (6) is converted to compound (4) as before i.e. with HBr or $BBr_3$.

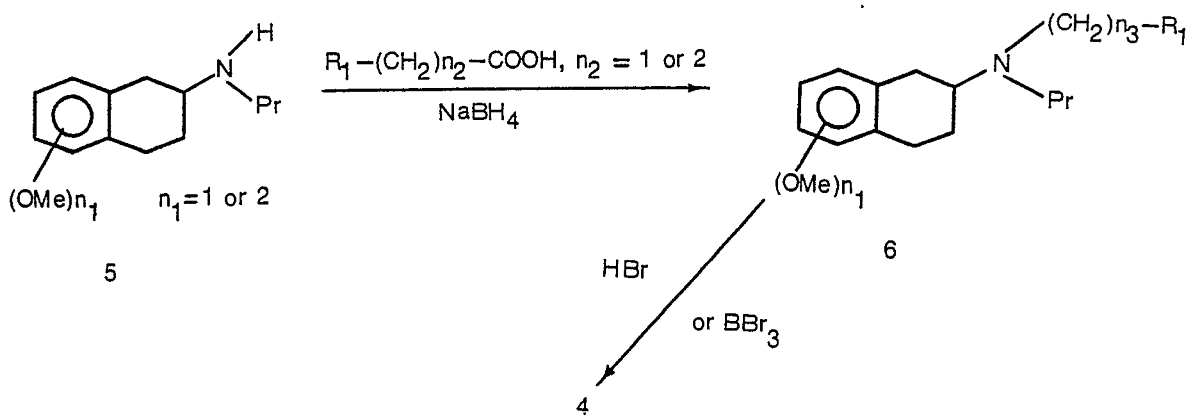


5

6

4

The prodrugs of these compounds where the —OH group is replaced by an ester, i.e. A is

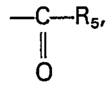

may be prepared by treating the compound with the desired corresponding acid chloride (Horn et al., J. Med. Chem. *25*, 993, 1982).

The compounds produced by the methods of this invention may be used in medical treatment, by administration of a therapeutically effective amount of the foregoing compounds. In general the daily dose can be from 0.01 mg/kg to 100 mg/kg per day and preferably from 0.1 mg/kg to 50 mg/kg per day, bearing in mind, of course, that in selecting the appropriate dosage in any specific case, consideration must be given to the patient's weight, general health, metabolism, age and other factors which influence response to the drug.

The compounds produced by the methods of the invention may also be utilized in pharmaceutical compositions in dosage unit form which comprise from about 1 mg to 100 mg of a compound of the above formula.

The pharmaceutical composition may be in a form suitable for oral use, for example, as tablets, aqueous or oily suspensions, dispersible powders or granules emulsions, hard or soft capsules, or syrups or elixers. Compositions intended for oral use may be prepared according to any method known in the art for the manufacture or pharmaceutical compositions and such compositions may contain one or more agents selected from the group consisting of sweetening agents, flavoring agents, coloring agents and preserving agents in order to provide a pharmaceutically elegant and palatable preparation. Tablets contain the active ingredient in admixture with non-toxic pharmaceutically acceptable excipients which are suitable for manufacture of tablets. These excipients may be, for example, inert diluents, for example calcium carbonate, sodium carbonate, lactose, calcium phosphate or sodium phosphate; granulating and disintegrating agents, for example maize starch, or alginic acid; binding agents, for example starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period.

Formulations for oral use may also be presented as hard gelatine capsules wherein the active ingredient is mixed with an inert solid diluent, for example calcium carbonate, calcium phosphate or kaolin, or as soft gelatine capsules wherein the active ingredient is mixed with an oil medium, for example arachis oil, liquid paraffin or olive oil.

Aqueous suspensions contain the active compound in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol, for example polyoxyethylene sorbitol monooleate, or condensation product of ethylene oxide with partial esters derived from fatty acids and hexitol anhydrides, for example polyoxyethylene sorbitan monooleate. The said aqueous suspensions may also contain one or more preservatives, for example ethyl, or n-propyl, p-hydroxy benzoate, one or more coloring agents, one or more flavoring agents and one or more sweetening agents, such as sucrose, saccharin, or sodium or calcim cyclamate.

Dispersible powders and granules suitable for preparation of an aqueous suspension by the addition of water provide the active ingredient in admixture with a dispersing or wetting agent, suspending agent and

one or more preservatives. Suitable dispersing or wetting agents and suspending agents are exemplified by those already mentioned above. Additional excipients, for example, sweetening, flavoring and coloring agents, may also be present.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable preparation, for example as a sterile injectable aqueous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol.

The pharmaceutical compositions may be tableted or otherwise formulated so that for every 100 parts by weight of the composition there are present between 5 and 95 parts by weight of the active ingredient and preferably between 25 and 85 parts by weight of the active ingredient. The dosage unit form will generally contain between about 1 mg and about 100 mg of the active ingredient of the formula stated above.

From the foregoing formulation discussion it is apparent that the compositions of this invention can be administered orally or parenterally. The term parenteral as used herein includes subcutaneous injection, intravenous, intramuscular, or intrasternal injection or fusion techniques.

The invention is further illustrated by the following Examples.

## Example I

Preparation of 7-hydroxy-2-(N-n-propyl-N-2-thienyl ethyl)-aminotetralin

This compound was prepared according to Method I.

7-Methoxy-2-tetralon (3.75 g) and β(2-thienyl) ethylamine (3.27 g) were dissolved in 50 ml dry toluene and p-toluenesulfonic acid (0.19 g) was added. This mixture was refluxed under an atmosphere of nitrogen for 2.5 hr with continual removal of water (Dean-Stark method). The toluene was then removed under reduced pressure and the residue was dissolved in a mixture of methanol (4 ml) and T.H.F. (60 ml). The pH of this mixture was adjusted to approximately 5 by addition of HCl-ether. Sodium cyanoborohydride (1.16 g) was then added and the mixture was stirred under nitrogen gas at room temperature for 2 hr. The solvents were then removed under reduced pressure and the residue was dissolved in ether (50 ml) and extracted with water (50 ml). This water layer was re-extracted with ether (50 ml). The combined ether fractions were washed with a saturated sodium chloride solution (50 ml) and the ether layer was then dried over anhydrous $MgSO_4$. Removal of the ether under reduced pressure yielded 6.96 g of the free base, which was then converted to a HCl salt (6.11 g, 90%). Recrystallization from ethanol/ether produced an analytical sample, m.p. 224—225°C.

The HCl salt (3.40 g) of the above secondary amine was dissolved in dichloromethane (40 ml) and triethylamine (2.52 g) was added. To this stirred solution at room temperature propionyl chloride (1.14 ml) was added in a dropwise fashion. During the whole of the above operation the temperature of the solution was kept at 5°C. This mixture was then stirred for a further 30 min after the completion of the addition of propionyl chloride. The reaction mixture was then filtered and the filtrate was evaporated under reduced pressure. Ether-HCl was then added and the resulting precipitated amine hydrochlorides were filtered off and discarded. The ether solution was then reduced to dryness to yield the intermediate amide (2.75 g, 76%).

The above amide was dissolved in tetrahydrofurane (25 ml) and this was added slowly to a mixture of $LiAlH_4$ (0.50 g) in tetrahydrofurane (40 ml) under nitrogen gas. After refluxing for 3 hr the mixture was allowed to cool then water (3.0 ml) and a 15% NaOH solution (2.75 ml) and then additional water (3 × 3 ml) was added. The solution was filtered off and the tetrahydrofurane fraction was reduced to dryness. The residue was dissolved in ether (50 ml) and extracted with water (20 ml). After drying over anhydrous $MgSO_4$ the ether layer was evaporated to yield the free base (2.06 g). Conversion to a HCl salt produced a white solid (1.80 g, 72%) which after recrystallization yielded an analytical sample, m.p. 159—160°C.

The above product (270 mg) was dissolved in dry dichloromethane and cooled to about −30°C and $BBr_3$ (7 ml) was added via a syringe. The mixture was stirred for 2 hr at this temperature and then for a further 2 hr at room temperature. Sufficient methanol was then added to produce a clear solution. It was then extracted with a saturated solution of $NaHCO_3$ (15 ml) and water (20 ml). The organic layer was separated and dried over anhydrous $MgSO_4$. Reduction to dryness and conversion to a HCl salt yielded 190 mg (73%) a white solid. The structure was confirmed by IR, MS, NMR and elemental analyses.

## Example II

Preparation of 5-hydroxy-2-(N-n-propyl-N-2-thienyl ethyl)-aminotetralin

This compound was prepared according to Method I.

5-Methoxy-2-tetralon (9.0 g, 51 mmol) and β(2-thienyl) ethylamine (7.8 g, 62 mmol) were dissolved in dry toluene (225 ml) and p-toluenesulfonic acid (0.19 g) was added. This mixture was refluxed under an atmosphere of nitrogen for 2.5 hr with continual removal of water (Dean-Stark method). The toluene was then removed under reduced pressure and the residue was dissolved in a mixture of methanol (15 ml) and tetrahydrofurane (225 ml). The pH of this mixture was then adjusted to approximately 5 by the addition of

HCl-ether. Sodium cyanoborohydride (2.0 g, 32 mmol) was then added and the mixture was stirred under nitrogen gas at room temperature for 2 hr. The solvents were then removed under reduced pressure and the residue was dissolved in ether (50 ml) and extracted with water (50 ml). This water layer was re-extracted with ether (50 ml). The combined ether fractions were washed with a saturated NaCl solution (50 ml) and the ether layer was dried over anhydrous $MgSO_4$. Removal of the ether yielded an oil which on distillation under reduced pressure (0.03 mm Hg 155—160°C) gave 9.8 g (67%) of the free base. Conversion to a HCl-salt gave an analytical sample m.p. 201—202°C.

The above free base (5.1 g, 17.8 mmol) was dissolved in dichloromethane (40 ml) containing triethylamine (2.0 g) and to this stirred solution at 5°C propionyl chloride (1.85 g, 20.0 mmol) was added in a dropwise manner. After the completion of the latter addition the mixture was stirred for a further 30 min. Most of the dichloromethane was then removed under reduced pressure and ether-HCl was added and the resulting precipitated amine hydrochlorides were filtered off and discarded. The ether solution was then reduced to dryness to yield the intermediate amide (5.9 g).

The amide (5.9 g, 17.2 mmol) was dissolved in dry tetrahydrofurane (50 ml) and this was added slowly to a suspension of $LiAlH_4$ (1.0 g, 26.3 mmol) in dry tetrahydrofurane (75 ml) under an atmosphere of nitrogen. After refluxing for 3 hr the mixture was allowed to cool and then water (5.0 ml) and a 15% NaOH solution (5.0 ml) and then additional water (3 × 5 ml) was added. The solution was filtered off and the tetrahydrofurane fraction was reduced to dryness. The residue was dissolved in ether (100 ml) and extracted with water (50 ml). After drying over anhydrous $MgSO_4$ the ether layer was reduced to dryness yielding the free base (5.0 g, 85%). An anlytical sample of the HCl salt had a m.p. of 148—150°C.

The above product (500 mg) was dissolved in dry dichloromethane and cooled to about −30°C and then 1N $BBr_3$ (7 ml) was added via a syringe. The mixture was stirred for 2 hr at this temperature and then for a further 2 hr at room temperature. Sufficient methanol was then added to produce a clear solution. It was then extracted with a saturated solution of $NaHCO_3$ (15 ml) and water (20 ml). The organic layer was dried over anhydrous $MgSO_4$. Reduction to dryness and conversion to a HCl salt yielded 300 mg (62.5%) of a white solid. The structure was confirmed by IR, MS, NMR and elemental analyses.

**Claims**

1. A compound having the structural formula

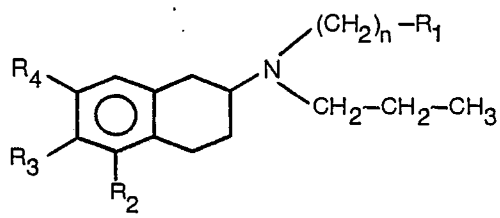

wherein $R_2$, $R_3$ and $R_4$ are each selected from H and OA; A is H or

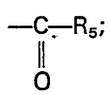

$R_5$ is selected from alkyl and aromatic residues; n is 2 or 3; and $R_1$ is selected from 3-pyridyl, 4-pyridyl,

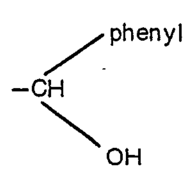 , 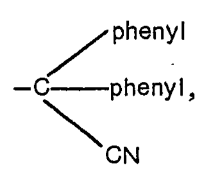

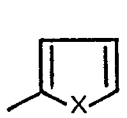 , 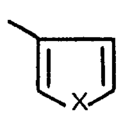 , 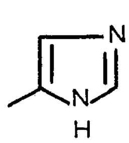 and 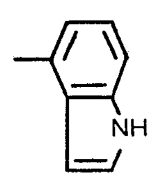

wherein X is S, O or NH, and a pharmaceutically acceptable salt thereof, with the proviso that at least one of the $R_2$, $R_3$ and $R_4$ is H, that at least one of $R_2$, $R_3$ and $R_4$ is not H and that $R_2$ and $R_4$ are not both OA.

2. The compound of claim 1 where $R_4$ is H and $R_2$ and $R_3$ are OH.

3. The compound of claim 1 where $R_2$ is H and $R_3$ and $R_4$ are OH.

4. The compound of claim 1 where $R_3$ and $R_4$ are H and $R_2$ is OH.

5. The compound of claim 1 where $R_2$ and $R_3$ are H and $R_4$ is OH.

6. The compound of claim 1 wherein n is 2.

7. 7-hydroxy-2-(N-n-propyl-N-2-thienyl ethyl)-aminotetralin.

8. 5-hydroxy-2-(N-n-propyl-N-2-thienyl ethyl)-aminotetralin.

9. The compound of claim 1, wherein $R_1$ is

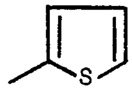
or
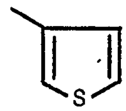

10. A process for the production of a compound according to claims 1 to 9 or a pharmaceutically acceptable salt thereof; comprising the steps of:

(a) condensing a β-tetralone of the formula

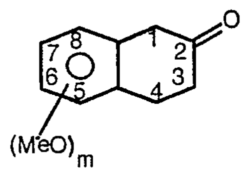

with a primary amine of the formula $R_{\emptyset}-(CH_2)_n-NH_2$ wherein m is 1 or 2, and if n is 3 then $R_{\emptyset}$ is $R_1$, and if n is 2 then $R_{\emptyset}$ is $CH_3$ or $R_1$;

(b) reducing the product of step (a) to form a secondary amine of the formula

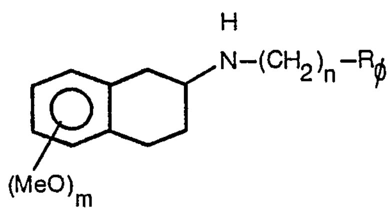

(c) reacting the product of step (b) either with a compound of the formula $R_6-(CH_2)_p-Y$ in the presence of a base or with a compound of the formula $R_6-(CH_2)_o-COOH$ in the presence of a reducing agent to form a tertiary amine, or with a compound of the formula

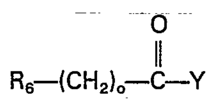

to form an amide and reducing the amide to a tertiary amine; in the above formulae Y is Cl, Br, I, tysolate, or mesylate; and if $R_{\emptyset}$ is $R_1$ then $R_6$ is $CH_3$, o is 1, and p is 2; and if $R_{\emptyset}$ is $CH_3$ then $R_6$ is $R_1$, o is 1 or 2, and p is 2 or 3; where the tertiary amine has the formula:

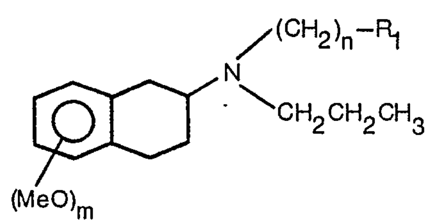
and

(d) demethylating the ether linkages of the tertiary amine to form the desired product.

11. The process of claim 10, further comprising the step of reacting the product of claim 10 with a carboxylic acid chloride to form an ester.

12. The process of claim 10, where n is 2 and $R_1$ is

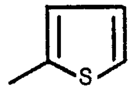
or
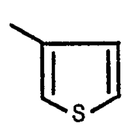

13. The process of claim 12, where m is 1 and the —OH group is in the 5 position.

14. The process of claim 12, where m is 2 and the —OH groups are in the 5 and 6 positions.

15. The process of claim 12, where m is 2 and the —OH groups are in the 6 and 7 positions.

**Patentansprüche**

1. Verbindung mit der Strukturformel

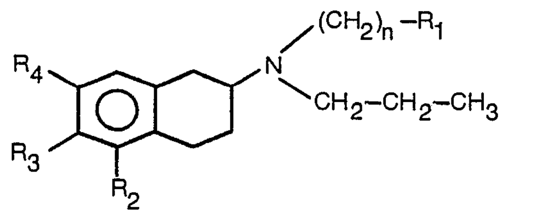

worin $R_2$, $R_3$ und $R_4$ jeweils aus H und OA ausgewählt sind, A für H oder

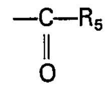

steht, $R_5$ aus Alkyl- und aromatischen Resten ausgewählt ist, n den Wert 2 oder 3 hat und $R_1$ aus 3-Pyridyl, 4-Pyridyl,

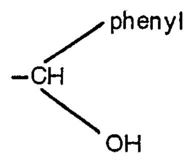
,
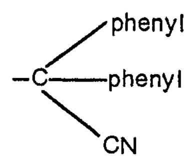
,

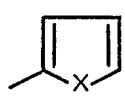
,
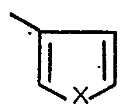
,
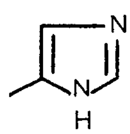
and
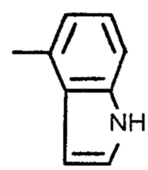

ausgewählt ist, worin X für S, O oder NH steht, und ein pharmazeutisch annehmbares Salz davon, mit der Maßgabe, daß mindestens eine der Gruppen $R_2$, $R_3$ und $R_4$ H ist, daß mindestens eine der Gruppen $R_2$, $R_3$ und $R_4$ nicht H ist und daß nicht beide Gruppen $R_2$ und $R_4$ OA sind.

2. Verbindung nach Anspruch 1, dadurch gekennzeichnet, daß $R_4$ für H und $R_2$ und $R_3$ für OH stehen.

3. Verbindung nach Anspruch 1, dadurch gekennzeichnet, daß $R_2$ für H und $R_3$ und $R_4$ für OH stehen.

4. Verbindung nach Anspruch 1, dadurch gekennzeichnet, daß $R_3$ und $R_4$ für H und $R_2$ für OH stehen.

5. Verbindung nach Anspruch 1, dadurch gekennzeichnet, daß $R_2$ und $R_3$ für H und $R_4$ für OH stehen.

6. Verbindung nach Anspruch 1, dadurch gekennzeichnet, daß n den Wert 2 hat.

7. 7-Hydroxy-2-(N-n-propyl-N-2-thienylethyl)-aminotetralin.

8. 5-Hydroxy-2-(N-n-propyl-N-2-thienylethyl)-aminotetralin.

9. Verbindung nach Anspruch 1, dadurch gekennzeichnet, daß $R_1$ für

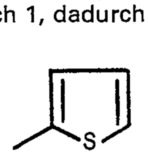
or
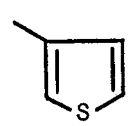
.

steht.

10. Verfahren zur Herstellung einer Verbindung nach den Ansprüchen 1 bis 9 oder eines pharmazeutisch annehmbaren Salzes davon, gekennzeichnet durch die Stufen:

(a) Kondensation eines β-Tetralons der Formel

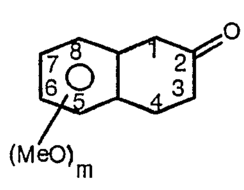

mit einem primären Amin der Formel $R_ø—(CH_2)_n—NH_2$, worin m den Wert 1 oder 2 hat und wenn n den Wert 3 hat, dann $R_ø$ $R_1$ ist, und wenn n den Wert 2 hat, dann $R_ø$ $CH_3$ oder $R_1$ ist;

(b) Reduktion des Produkts der Stufe (a) zur Bildung eines sekundären Amins der Formel

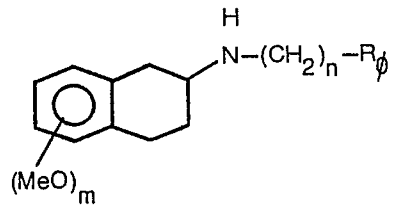

(c) Umsetzung des Produkts der Stufe (b) entweder mit einer Verbindung der Formel $R_6-(CH_2)_p-Y$ in Gegenwart einer Base oder mit einer Verbindung der Formel $R_6-(CH_2)_o-COOH$ in Gegenwart eines Reduktionsmittels, um ein tertiäres Amin zu bilden, oder mit einer Verbindung der Formel

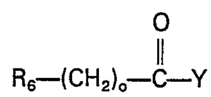

um ein Amid zu bilden, und Reduktion des Amids zu einem tertiären Amin, wobei in den obigen Formeln Y für Cl, Br, I, Tysolat oder Mesylat steht und wenn $R_ø$ $R_1$ ist, dann $R_6$ $CH_3$ ist, o den Wert 1 hat und p den Wert 2 hat und wenn $R_ø$ $CH_3$ ist, dann $R_6$ $R_1$ ist, o den Wert 1 oder 2 hat und p den Wert 2 oder 3 hat, wobei das tertiäre Amin die formel

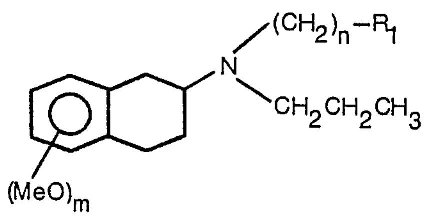

and

aufweist; und

(d) Demethylierung der Etherbindungen des tertiären Amins, um das gewünschte Produkt zu bilden.

11. Verfahren nach Anspruch 10, gekennzeichnet durch die weitere Stufe der Umsetzung des Produkts nach Anspruch 10 mit einem Carbonsäurechlorid, um einen Ester zu bilden.

12. Verfahren nach Anspruch 10, dadurch gekennzeichnet, daß n den Wert 2 hat und $R_1$ für

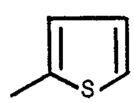 or 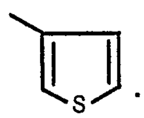

steht.

13. Verfahren nach Anspruch 12, dadurch gekennzeichnet, daß m den Wert 1 hat und daß sich die —OH-Gruppe in der 5-Stellung befindet.

14. Verfahren nach Anspruch 12, dadurch gekennzeichnet, daß m den Wert 2 hat und daß sich die —OH-Gruppen in den 5- und 6-Stellungen befinden.

15. Verfahren nach Anspruch 12, dadurch gekennzeichnet, daß m den Wert 2 hat und daß sich die —OH-Gruppen in den 6- und 7-Stellungen befinden.

## Revendications

1. Composé ayant la formule structurale suivante:

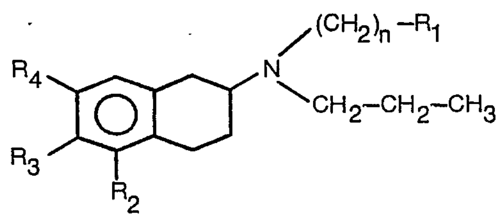

dans laquelle $R_2$, $R_3$ et $R_4$ sont respectivement choisis parmi H et OA; A est H ou

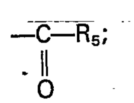

$R_5$ est parmi des résidues alkyles et aromatiques; n est 2 ou 3; et $R_1$ est choisi dans le groupe comprenant des radicaux 3-pyridyle, 4-pyridyle,

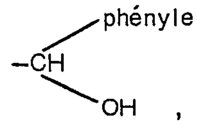
,
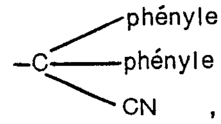
,

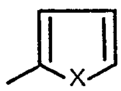
,
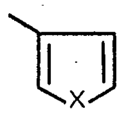
,
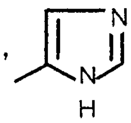
et
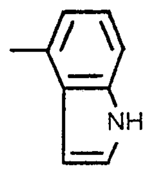

dans lesquels X est S, O ou NH, et un sel de celui-ci acceptable du point de vue pharmaceutique, à condition qu'au moins l'un des $R_2$, $R_3$ et $R_4$ soit H, qu'au moins l'un des $R_2$, $R_3$ et $R_4$ ne soit pas H et que $R_2$ et $R_4$ ne soient pas tous les deux un groupe OA.

2. Composé suivant la revendication 1, caractérisé en ce que $R_4$ est H et que $R_2$ et $R_3$ sont OH.

3. Composé suivant la revendication 1, caractérisé en ce que $R_2$ est H et que $R_3$ et $R_4$ sont OH.

4. Composé suivant la revendication 1, caractérisé en ce que $R_3$ et $R_4$ sont H et $R_2$ est OH.

5. Composé suivant la revendication 1, caractérisé en ce que $R_2$ et $R_3$ sont H et $R_4$ est OH.

6. Composé suivant la revendication 1, caractérisé en ce que n est 2.

7. Composé suivant la revendication 1, caractérisé en ce qu'il s'agit de la 7-hydroxy-2-(N-n-propyl-N-2-thiényléthyl)aminotétraline.

8. Composé suivant la revendication 1, caractérisé en ce qu'il s'agit de la 5-hydroxy-2-(N-n-propyl-N-2-thiényléthyl)aminotétraline.

9. Composé suivant la revendication 1, caractérisé en ce que $R_1$ est un groupe

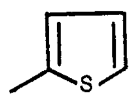
or
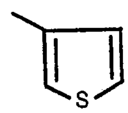
.

10. Procédé pour la production d'un composé suivant les revendications 1 à 9 ou d'un sel de celui-ci acceptable du point de vue pharmaceutique, caractérisé en ce qu'il comprend les étapes de:

(a) condensation d'une β-tétralone de formule

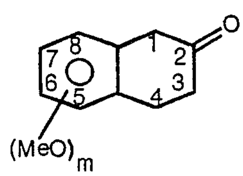

avec une amine primaire de formule $R-(CH_2)_n-NH_2$, dans laquelle m est 1 ou 2, et si n est 3 alors R est $R_1$, et si n est 2, alors R est $CH_3$ ou $R_1$;

(b) réduction du produit de l'étape (a) pour former une amine secondiare de formule

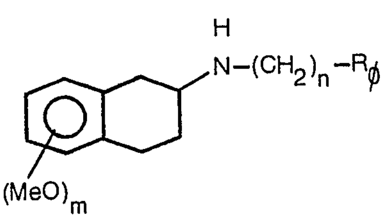

(c) réaction du produit de l'étape (b) avec un composé de formule $R_6-(CH_2)_p-Y$ en présence d'une base, ou avec un composé de formule $R_6-(CH_2)_o-COOH$ en présence d'un agent réducteur pour former une amine tertiaire, ou avec un composé de formule

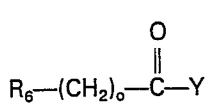

pour former un amide et réduction de l'amide en une amine tertiaire; dans les formules ci-dessus, Y est Cl,

Br, I, tosylate ou mésylate; et si R est $R_1$, alors $R_6$ est $CH_3$, o est 1 et p est 2; et si R est $CH_3$ alors $R_6$ est $R_1$, o est 1 ou 2 et p est 2 ou 3; l'amine tertiaire ayant la formule:

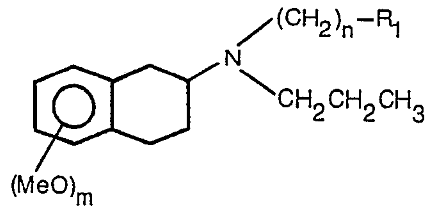

and

(d) déméthylation des liaisons éther de l'amine tertiaire pour former le produit désiré.

11. Procédé suivant la revendication 10, caractérisé en ce qu'il comprend de plus l'étape de réaction du produit de la revendication 10 avec un chlorure d'acide carboxylique pour former un ester.

12. Procédé suivant la revendication 10, caractérisé en ce que n est 2 et que $R_1$ est un groupe

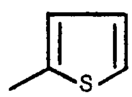

or

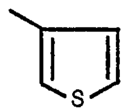

.

13. Procédé suivant la revendication 12, caractérisé en ce que m est 1 et que le groupe —OH est en position 5.

14. Procédé suivant la revendication 12, caractérisé en ce que m est 2 et que les groupes —OH sont en positions 5 et 6.

15. Procédé suivant la revendication 12, caractérisé en ce que m est 2 et que les groupes —OH sont en positions 6 et 7.